# EUROPEAN PATENT SPECIFICATION

(11) **EP 3 556 855 B1**
(45) Date of publication and mention of the grant of the patent: **14.01.2026**
(21) Application number: 17881391.1
(22) Date of filing: 06.12.2017
(51) Int. Cl.: C07K 14/705, A61P 35/00, C07K 14/52, C07K 14/725, C07K 16/28, A61K 40/11, A61K 40/31, A61K 40/42, C12N 5/0783

(54) **CHIMERIC ANTIGEN RECEPTOR GENE-MODIFIED LYMPHOCYTE HAVING CYTOCIDAL EFFECT**
GENMODIFIZIERTES LYMPHOZYT MIT CHIMÄREM ANTIGENREZEPTOR MIT ZELLZERSTÖRENDER WIRKUNG
LYMPHOCYTE GÉNÉTIQUEMENT MODIFIÉ DE RÉCEPTEUR D'ANTIGÈNE CHIMÉRIQUE POSSÉDANT UN EFFET DE CYTOTOXICITÉ

(30) Priority: 14.12.2016 JP 2016242054
(43) Date of publication of application: 23.10.2019
(73) Proprietor: Shinshu University, Matsumoto City, Nagano 390-8621 (JP); Kyoto Prefectural Public University Corporation, Kyoto-shi, Kyoto 602-8566 (JP)
(72) Inventor: HOSOI, Hajime, Kyoto-shi, Kyoto 602-8566 (JP); IEHARA, Tomoko, Kyoto-shi, Kyoto 602-8566 (JP); YAGYU, Shigeki, Kyoto-shi, Kyoto 602-8566 (JP); NAKAZAWA, Yozo, Matsumoto City, Nagano 390-8621 (JP)
(74) Representative: Witthoff Jaekel Steinecke Patentanwälte PartG mbB
(86) International application number: PCT/JP2017/043729
(87) International publication number: WO 2018/110374

(56) References cited:
- JP-A- 2008 545 375
- JP-A- 2011 256 179
- JP-A- 2013 189 443
- JP-A- 2013 536 806
- MICHAEL H. KERSHAW ET AL: "Gene-engineered T cells for cancer therapy", NATURE REVIEWS. CANCER, vol. 13, no. 8, 1 August 2013 (2013-08-01), GB, pages 525 - 541, XP055543320, ISSN: 1474-175X, DOI: 10.1038/nrc3565
- NAKAGAWA, TAKESHI ET AL.: "Development of next-generation adoptive immunotherapy using cytotoxic T-lymphocyte(CTL) expressing chimeric antigen-receptor(CAR)", DRUG DELI VERY SYSTEM, vol. 28, no. 1, 2013, pages 35 - 44, XP055496872, ISSN: 0913-5006
- NAKAZAWA, YOZO: "Gene -modified T- cell Therapy Using Chimeric Antigen Receptor", THE SHINSHU MEDICAL JOURNAL, vol. 61, no. 4, 2013, pages 197 - 203, XP055516593, ISSN: 0037-3826
- SAITO, S. ET AL.: "Anti-leukemic potency of piggyBac-mediated CD 19-specific T cells against refractory Philadelphia chromosome-positive acute lymphoblastic leukemia", CYTOTHERAPY, vol. 16, September 2014 (2014-09-01), pages 1257 - 1269, XP055181626, ISSN: 1465-3249
- LV , J. ET AL.: "EphB4 promotes the proliferation, invasion, and angiogenesis of human colorectal cancer", EXPERIMENTAL AND MOLECULAR PATHOLOGY, vol. 100, no. 3, 2016, pages 402 - 408, XP029572497, ISSN: 0014-4800
- NOREN, N. K. ET AL.: "The EphB4 receptor suppresses breast cancer cell tumorigenicity through an Abl-Crk pathway", NATURE CELL BIOLOGY, vol. 8, no. 8, 23 July 2006 (2006-07-23), pages 815 - 825, XP055496904, ISSN: 1465-7392

## Description

### [Technical Field]

The present invention relates to a gene-modified lymphocyte that expresses a chimeric antigen receptor (CAR) (CAR gene-introduced lymphocyte). In particular, the present invention relates to a chimeric antigen receptor gene-modified lymphocyte capable of exerting a cytocidal effect on a highly EPHB4 receptor (Ephrin type-B receptor 4) expressing tumor, use thereof, and the like.

### [Background Art]

In order to recover the tumor immune mechanism of cancer patients, there has been developed, in recent years, treatment with gene-modified chimeric antigen receptor (CAR) T cells (CAR-T therapy) in which genetic modification is added to T cell receptors (TCRs) possessed by cytotoxic T cells (CTLs) to allow the CTLs to directly and selectively recognize tumor cells, thereby exerting an antitumor effect (see, for example, NPL 1). CAR is a generic term for proteins having a protein that specifically recognizes a tumor antigen (usually, a single-chain antibody (scFv) having an antibody variable region modified to a single-chain amino acid sequence is used) on the N-terminal side and a T cell receptor ζ chain on the C-terminal side. CAR-expressing T cells (CAR-T cells) recognize the tumor antigen in the extracellular domain, and subsequently transmit the signal into the T cells through the ζ chain to activate the T cells, and release cytocidal factors such as perforin and granzymes to exert an antitumor effect (see, for example, NPL 1).

Cancer treatment using CAR-T cells has already been applied as a clinical trial. In the hematologic tumor area, phase 3 clinical trials were conducted for CD19-positive B-lymphoid tumors. Specifically, the trials targeted for patients with relapsed acute lymphocytic leukemia. As a result of introducing CD19-specific CAR genes into T cells collected from the patients, culturing and proliferating the cells, and infusing them into the patients' bodies, molecular biological remission in the bone marrow was obtained in all 5 cases (see, for example, NPL 2).

### [Citation List]

### [Non Patent Literature]

[NPL 1] Eshhar Z, Waks T, Gross G, Schindler DG. Specific activation and targeting of cytotoxic lymphocytes through chimeric single-chains consistent with antibody-binding domains and the gamma or zeta subunits of the immunoglobulins and T-cell receptors. Proc Natl Acad Sci USA. 1993; 90: 720-724.
[NPL 2] Brentjens RJ, Davila ML, Riviere I, Park J, Wang X, Cowell LG, Bartido S, Stefanski J, Taylor C, Olszewska M, Borquez-Ojeda O, Qu J, Wasielewska T, He Q, Bernal Y, Rijo IV, Hedvat C, Kobos R, Curran K, Steinherz P, Jurcic J, Rosenblat T, Maslak P, Frattini M, Sadelain M. CD19-targeted T cells rapidly induce molecular remissions in adults with chemotherapy-refractory acute Leukemia. Sci Transl Med. 2013; 5: 177ra38.
[NPL 3] Louis CU, Savoldo B, Dotti G, Pule M, Yvon E, Myers GD, Rossig C, Russell HV, Diouf O, Liu E, Liu H, Wu MF, Gee AP, Mei Z, Rooney CM, Heslop HE, Brenner MK. Antitumor activity and long-term fate of chimeric antigen receptor-positive T cells in patients with neuroblastoma. Blood. 2011; 118: 6050-6056.
[NPL 4] Ahmed N, Brawley VS, Hegde M, Robertson C, Ghazi A, Gerken C, Liu E, Dakhova O, Ashori A, Corder A, Gray T, Wu MF, Liu H, Hicks J, Rainusso N, Dotti G, Mei Z, Grilley B, Gee A, Rooney CM, Brenner MK, Heslop HE, Wels WS, Wang LL, Anderson P, Gottschalk S. Human Epidermal Growth Factor Receptor 2 (HER2)-Specific Chimeric Antigen Receptor-Modified T Cells for the Immunotherapy of HER2-Positive Sarcoma. J Clin Oncol. 2015; 33: 1688-1696.
[NPL 5] Abate-Daga D, Davila ML CAR models: next-generation CAR modifications for enhanced T-cell function. MoI Ther-Oncolytics 2016; 3; 16014
[NPL 6] Gerety SS, Wang HU, Chen ZF, Anderson DJ. Symmetrical mutant phenotypes of the receptor EphB4 and its specific transmembrane ligand ephrin-B2 in cardiovascular development. Mol. Cell. 1999; 4; 403-14.
[NPL 7] Lv J, Xia Q, Wang J, Shen Q, Zhang J, Zhou X. EphB4 promotes the proliferation, invasion, and angiogenesis of human colorectal cancer. Exp Mol Pathol. 2016; 100; 402-8.
[NPL 8] Pradeep S, Huang J, Mora EM, Nick AM, Cho MS, Wu SY, Noh K, Pecot CV, Rupaimoole R, Stein MA, Brock S, Wen Y, Xiong C, Gharpure K, Hansen JM, Nagaraja AS, Previs RA, Vivas-Mejia P, Han HD, Hu W, Mangala LS, Zand B, Stagg LJ, Ladbury JE, Ozpolat B, Alpay SN, Nishimura M, Stone RL, Matsuo K, Armaiz-Pena GN, Dalton HJ, Danes C, Goodman B, Rodriguez-Aguayo C, Kruger C, Schneider A, Haghpeykar S, Jaladurgam P, Hung MC, Coleman RL, Liu J, Li C, Urbauer D, Lopez-Berestein G, Jackson DB, Sood AK. Erythropoietin Stimulates Tumor Growth via EphB4. Cancer Cell. 2015; 28; 610-22.
[NPL 9] Ferguson BD, Tan YH, Kantet RS, Liu R, Gayed MJ, Vokes EE, Ferguson MK, Iafrate AJ, Gill PS, Salgia R. Novel EPHB4 Receptor Tyrosine Kinase Mutations and Kinomic Pathway Analysis in Lung Cancer. Sci Rep. 2015; 5; 10641.
[NPL 10] Becerikli M, Merwart B, Lam MC, Suppelna P, Rittig A, Mirmohammedsadegh A, Stricker I, Theiss C, Singer BB, Jacobsen F, Steinstraesser L. EPHB4 tyrosine-kinase receptor expression and biological significance in soft tissue sarcoma. Int J Cancer. 2015; 136; 1781-91.
[NPL 11] Mertens-Walker I, Lisle JE, Nyberg WA, Stephens CR, Burke L, Rutkowski R, Herington AC, Stephenson SA. EphB4 localises to the nucleus of prostate cancer cells. Exp Cell Res. 2015; 333; 105-15.
[NPL 12] Ferguson BD, Tretiakova MS, Lingen MW, Gill PS, Salgia R. Expression of the EPHB4 receptor tyrosine kinase in head and neck and renal malignancies-implications for solid tumors and potential for therapeutic inhibition. Growth Factors. 2014; 32; 202-6.
[NPL 13] Aslam MI, Abraham J, Mansoor A, Druker BJ, Tyner JW, Keller C. PDGFRβ reverses EphB4 signaling in alveolar rhabdomyosarcoma. Proc Natl Acad Sci U S A. 2014; 111; 6383-8.
[NPL 14] Schmitt F, Nguyen PH, Gupta N, Mayer D. Eph receptor B4 is a regulator of estrogen receptor alpha in breast cancer cells. J Recept Signal Transduct Res. 2013; 33; 244-8.
[NPL 15] Ferguson BD, Liu R, Rolle CE, Tan YH, Krasnoperov V, Kanteti R, Tretiakova MS, Cervantes GM, Hasina R, Hseu RD, Iafrate AJ, Karrison T, Ferguson MK, Husain AN, Faoro L, Vokes EE, Gill PS, Salgia R. The EphB4 receptor tyrosine kinase promotes lung cancer growth: a potential novel therapeutic target. PLoS One. 2013; 8; e67668.
[NPL 16] Liu R, Ferguson BD, Zhou Y, Naga K, Salgia R, Gill PS, Krasnoperov V. EphB4 as a therapeutic target in mesothelioma. BMC Cancer. 2013; 13; 269.
[NPL 17] Li M, Zhao Z. Clinical implications of EphB4 receptor expression in pancreatic cancer. MoI Biol Rep. 2013; 40; 1735-41.
[NPL 18] Hasina R, Mollberg N, Kawada I, Mutreja K, Kanade G, Yala S, Surati M, Liu R, Li X, Zhou Y, Ferguson BD, Nallasura V, Cohen KS, Hyjek E, Mueller J, Kanaseti R, El Hashani E, Kane D, Shimada Y, Lingen MW, Husain AN, Posner MC, Waxman I, Villaflor VM, Ferguson MK, Varticovski L, Vokes EE, Gill P, Salgia R. Critical role for the receptor tyrosinese kinase EPHB4 in esophageal cancers. Cancer Res. 2013; 73; 184-94.
[NPL 19] Rutkowski R, Mertens-Walker I, Lisle JE, Herington AC, Stephenson SA. Evidence for a dual function of EphB4 as tumor promoter and suppressor regulated by the absence or presence of the ephrin-B2 ligand. Int J Cancer; 2012; 131; E614-24.
[NPL 20] Noren NK1, Foos G, Hauser CA, Pasquale EB. The EphB4 receptor suppresses breast cancer cells tumorigenicity through an Abl-Crk pathway. Nat Cell Biol. 2006; 8; 815-25. The authors describe that wild typical EphB4 receptor can behave as a tumour suppressor when stimulated by its ligand.
[NPL 21] Aitsebaomo J1, Portbury AL, Schisler JC, Patterson C. Brothers and sisters: molecular insights into arterial-venous heterogeneity. Circ Res. 2008; 103; 929-39.
[NPL 22] Boyd AW, Bartlett PF, Lackmann M. Therapeutic targeting of EPH receptors and their ligands. Nat Rev Drug Discovery 2014; 1339-62.
[NPL 23] Saito et al., Anti-leukemic potency of piggyBac-mediated CD19-specific T cells against refractory Philadelphia chromosome-positive acute lymphoblastic leukemia. Cytotherapy. 2014 September; 16(9): 1257-1269. Saito et al. describe T cells engineered to express a CD19-specific chimeric antigen receptor (CAR) as a potential treatment option for Philadelphia chromosome-positive acute lymphoblastic leukemia.

### [Summary of Invention]

### [Technical Problem]

Although CAR-T cell therapies for neuroblastoma and osteosarcoma are being developed in the solid tumor area (NPL 3 and NPL 4), sufficient research has not been made yet, nor has clinical application been conducted. Therefore, an object of the present invention is to provide a therapeutic strategy in the solid tumor area and a means useful therefor to further advance the clinical application of CAR therapy.

### [Solution to Problem]

In order to cause CAR-T cells to specifically and potently act on tumor cells, (1) identification of cancer antigens which are highly expressed only in tumors and can be targeted by CAR-T cells, (2) identification and cloning of molecules specifically binding to the cancer antigens are important (see, for example, NPL 5). The present inventors have focused on an EPHB4 receptor in advancing the study in consideration of this point. EPHB4 is a member of the Eph family of receptor tyrosine kinases. In embryonic development, the binding, to EPHB4, of EphrinB2 as its ligand plays an important role in the regulation of cell adhesion and cell movement and in the development of blood vessels (see NPL 6). Furthermore, the inappropriate function of the Eph receptor may cause malignancy, as tissue breakdown and abnormal cell adhesion, movement and survival are features that are shown at advanced stages of cancer. In fact, it has been reported that EPHB4 is highly expressed in various cancers such as lung cancer, bowel cancer, malignant mesothelioma, esophagus cancer, breast cancer, and rhabdomyosarcoma, and is involved in malignant transformation of cancer. (See NPLs 7 to 20). In addition, it has been shown that the proliferation of these tumor cells is markedly suppressed by the ephrinB2-Fc molecule that selectively blocks the function of EPHB4 (see NPL 20). Furthermore, although EPHB4 is partially expressed weakly in vascular endothelial cells, the expression in other normal tissues is extremely low (see NPL 21). In addition, no significant adverse events were observed in clinical trials involving application of an EphB4 inhibitor, EPHB4-HSA, to malignant tumor patients (see NPL 22). Interestingly, it has been revealed that the activation of EPHB4 in cancer malignant transformation occurs in a ligand-independent manner, and that the ligand-dependent activation of EPHB4 (activation of EPHB4 by binding of EphrinB2 to EPHB4) in cancer cells induces cell death in cancer cells (see NPLs 13 and 20).

According to the above observation, EPHB4 is considered to be a promising cancer antigen, i.e., a "therapeutic target", in tumors that highly express EPHB4. Therefore, the present inventors have arrived at the idea that tumor cells that highly express EPHB4 can be specifically attacked by constructing a gene-modified T cell that specifically recognizes EPHB4. Since EPHB4 is a receptor, its ligand EphrinB2 can specifically and potently bind to EPHB4. It is considered that the incorporation of the binding site (extracellular domain) to EPHB4 in EphrinB2 into a chimeric antigen receptor (CAR) can construct a gene-modified T cell which specifically recognizes an EPHB4 expressing tumor cell and having a cytocidal effect. Furthermore, it is considered that, since only the binding between a CAR having an EphrinB2 extracellular domain and EPHB4 alone is also expected to induce cell death, the CAR has a therapeutic effect higher than that of a CAR using a conventional antibody variable region and is more ideal.

Based on the above idea, an EPHB4-specific CAR vector (EPHB4-CAR vector) in which a gene encoding EphrinB2 (EFNB2) extracellular domain is inserted into a CAR expression vector has been constructed to prepare a gene-modified T cell (EPHB4-CAR-T cell). It is considered that the use of the EphrinB2 extracellular domain as an EPHB4 recognition site of a CAR can be expected to provide both of a cell death induction effect on cancer cells by EphrinB2-EPHB4 binding and a cytocidal effect of the CAR-T cell, which are not observed in conventional single-chain antibody type CARs. In fact, as a result of study on the properties and effects of EPHB4-CAR-T cells, it has been proved that a tumor highly expressing EPHB4 can be specifically killed. That is, the effectiveness of the strategy created by the present inventors has been confirmed by experiments.

Based on the above results, the following inventions are provided.
[1] An EPHB4 receptor-specific chimeric antigen receptor comprising an extracellular domain including an EphrinB2 extracellular domain, a transmembrane domain, and an intracellular signal domain for the effector function of immunocytes.
[2] The EPHB4 receptor-specific chimeric antigen receptor according to [1], wherein the EphrinB2 extracellular domain comprises an amino acid sequence of SEQ ID NO: 1.
[3] The EPHB4 receptor-specific chimeric antigen receptor according to [1] or [2], wherein the intracellular signal domain includes the intracellular domain of a costimulatory molecule and CD3ζ.
[4] The EPHB4 receptor-specific chimeric antigen receptor according to [3], wherein the costimulatory molecule is CD28.
[5] The EPHB4 receptor-specific chimeric antigen receptor according to any one of [1] to [4], comprising a spacer domain between the extracellular domain and the transmembrane domain.
[6] A gene encoding the EPHB4 receptor-specific chimeric antigen receptor according to any one of [1] to [5].
[7] A method for preparing a gene-modified lymphocyte expressing a chimeric antigen receptor, comprising a step of introducing the gene according to [6] into a target cell in vitro.
[8] The preparation method according to [7], wherein the introduction of the gene is carried out by a transposon method.
[9] The preparation method according to [8], wherein the transposon method is the piggyBac transposon method.
[10] The preparation method according to any one of [7] to [9], wherein the target cell is a T cell.
[11] A gene-modified lymphocyte expressing the EPHB4 receptor-specific chimeric antigen receptor according to any one of [1] to [5].
[12] The gene-modified lymphocyte according to [11], wherein the lymphocyte is a T cell.
[13] A cell preparation comprising a therapeutically effective amount of the gene-modified lymphocyte according to [11] or [12].
[14] The cell preparation according to [13] or the gene-modified lymphocyte according to [11] or [12]for use in a method of treatment of tumor or cancer selected from the group consisting of rhabdomyosarcoma, lung cancer, bowel cancer, malignant mesothelioma, esophagus cancer, breast cancer, ovarian cancer, melanoma, and head and neck cancer.
[15] An expression cassette comprising a promoter and the gene according to [6] under the control of the promoter.
[16] A vector having the expression cassette according to [15].
[17] The vector according to [16], wherein the vector has a structure that the expression cassette is sandwiched between a pair of transposon inverted repeat sequences.
[18] A kit for preparing a gene-modified lymphocyte expressing an EPHB4 receptor-specific chimeric antigen receptor, the kit comprising the vector according to [17] and a transposase expression vector.
[19] The preparation kit according to [18], wherein the transposase is piggyBac transposase.

### [Brief Description of Drawings]

[FIG. 1] Structure of a CAR expression piggyBac transposon vector. Left: CD19-CAR expression vector (pIRII-CD19-CAR), right: EPHB4-CAR expression vector (pIRII-EPHB4-CAR).
[FIG. 2] Expression rate of EPHB4-CAR on T cells. The CAR gene introduction rate of T cells on Day 15 after gene introduction operation using an EPHB4-CAR expression vector and a pCMV-piggyBac vector was measured by flow cytometry.
[FIG. 3] Tumor cell proliferation inhibitory effect by EPHB4-CAR-T cells. CAR-T cells (CD19-CAR-T cells or EPHB4-CAR-T cells) and tumor cells (Rh30 cells or Raji cells) were co-cultured in a cell ratio of 2:1. On Day 0 and Day 3 of co-culture, the number of surviving tumor cells was measured by flow cytometry. The same experiment was repeated three times to calculate an average value. Upper: Proliferation inhibitory effect on Rh30 cells. Lower: Proliferation inhibitory effect on Raji cells.
[FIG. 4] IFNγ production ability of EPHB4-CAR-T cells. CAR-T cells (CD19-CAR-T cells or EPHB4-CAR-T cells) and Rh30 cells were co-cultured in a cell ratio of 2:1. IFNγ released into the culture supernatant was quantified by the ELISA method on Day 0 and Day 3 of co-culture. The same experiment was repeated three times to calculate an average value.
[FIG. 5] Results of an experiment (live imaging) using mouse models. The tumor size was assessed every 7 days (right) by live imaging (left) after injection of CAR-T cells into tumor-bearing mice.

### [Description of Embodiments]

The present invention relates to the embodiments as characterized in the claims.

### 1. Chimeric antigen receptor

A first aspect of the present invention relates to a chimeric antigen receptor specific to EPHB4 (Ephrin type-B receptor 4) receptor (chimeric antigen receptor is referred to as "CAR" in accordance with customary practice). The CAR of the present invention targets EPHB4 and shows a strong binding specific to EPHB4 receptor (binding specificity). In order to exert its unique function, the CAR of the present invention has a characteristic structure. Specifically, it has an extracellular domain including an EphrinB2 extracellular domain, a transmembrane domain, and an intracellular signal domain for the effector function of immunocytes.

### (a) Extracellular domain

Common CARs utilize single-chain antibodies (scFv) that specifically recognize a target to obtain antigen specificity. In contrast, the CAR of the present invention utilizes an extracellular domain of EphrinB2 protein, which is a natural ligand of the target EPHB4 receptor, for antigen recognition. The amino acid sequence of the extracellular domain of EphrinB2 protein is shown in SEQ ID NO: 1. Typically, the extracellular domain of the CAR of the present invention is constituted by a polypeptide chain consisting of the amino acid sequence. However, the extracellular domain of the CAR of the present invention may contain a portion other than the extracellular domain of EphrinB2 protein, or alternatively, may be composed of a part of the extracellular domain of EphrinB2 protein as long as it exhibits the function, i.e., specificity for EPHB4. In addition, as long as the specificity for EPHB4 is not impaired, a part of the above amino acid sequence may be modified. The modification herein can occur by deletion, substitution, addition, and the like of amino acid residues constituting the amino acid sequence. The number of amino acids to be modified is, for example, 50 or less, preferably 25 or less, more preferably 15 or less, still more preferably 10 or less, and most preferably 5 or less.

### (b) Transmembrane domain

The transmembrane domain intervenes between the extracellular domain and intracellular signal domain. Examples of the transmembrane domain used herein include CD28, CD3ε, CD8α, CD3, CD4, and 4-1BB. Alternatively, a transmembrane domain composed of an artificially constructed polypeptide may be used.

### (c) Intracellular signal domain

The intracellular signal domain transmits the signals necessary for exertion of the effector function of immunocytes. More specifically, when the extracellular domain binds with the target antigen, an intracellular signal domain capable of transmitting the signals necessary for activation of immunocytes are used. The intracellular signal domain includes the domain for transmitting the signals through the TCR complex (for convenience, referred to as "the first domain"), and the domain for transmitting the costimulatory signals (for convenience, referred to as "the second domain"). As the first domain, CD3ξ or other intracellular domains such as FcεRIγ may be used. The use of CD3ξ is preferred. As the second domain, the intracellular domain of a costimulatory molecule is used. Examples of the costimulatory molecule include CD28, 4-1BB (CD137), CD2, CD4, CD5, CD134, OX-40, and ICOS. The use of the intracellular domain of CD28 or 4-1BB is preferred.

The linking form of the first and second domains is not particularly limited, and preferably the second domain is disposed on the transmembrane domain side, because it is known that co-stimulation was strongly transmitted when CD3ξ was linked distally in a past case. The same or different kinds of plural intracellular domains may be linked in tandem to form the first domain. The same holds true for the second domain.

The first and second domains may be directly linked, or a linker may intervene between them. The linker may be, for example, a peptide linker. The peptide linker is composed of peptides which are linear chains of amino acids. The structure and characteristics of the peptide linker are as described above. However, the linker used herein may be composed solely of glycine. The length of the linker is not particularly limited. For example, a linker composed of 2 to 15 amino acid residues may be used.

### (d) Other elements

A leader sequence (signal peptide) is used to promote CAR secretion. For example, the leader sequence of the GM-CSF receptor may be used. In addition, the structure is preferably composed of an extracellular domain and a transmembrane domain linked together through a spacer domain. More specifically, the CAR according to a preferred embodiment contains a spacer domain between the extracellular domain and transmembrane domain. The spacer domain is used for promoting linking between the CAR and target antigen. For example, the Fc fragment of a human IgG (for example, human IgG1 or human IgG4) may be used as the spacer domain. Alternatively, a part of the extracellular domain of CD28 or a part of the extracellular domain of CD8α may be used as the spacer domain. A spacer domain may be placed between the transmembrane domain and intracellular signal domain.

There are some reports on the experiments and clinical studies using CARs (for example, Rossig C, et al. Mol Ther 10: 5-18, 2004; Dotti G, et al. Hum Gene Ther 20: 1229-1239, 2009; Ngo MC, et al. Hum Mol Genet 20 (R1): R93-99, 2011; Ahmed N, et al. Mol Ther 17: 1779-1787, 2009; Pule MA, et al. Nat Med 14: 1264-1270, 2008; Louis CU, et al. Blood 118: 6050-6056, 2011; Kochenderfer JN, et al. Blood 116: 4099-4102, 2010; Kochenderfer JN, et al. Blood 119: 2709-2720, 2012; Porter DL, et al. N Engl J Med 365: 725-733, 2011; Kalos M, et al. Sci Transl Med 3: 95ra73,2011; Brentjens RJ, et al. Blood 118: 4817-4828, 2011; and Brentjens RJ, et al. Sci Transl Med 5: 177 ra38, 2013), and the CARs in the present invention may be constructed with reference to these reports.

### 2. Gene encoding chimeric antigen receptor (CAR) and use thereof

A second aspect of the present invention relates to a CAR-encoding gene (hereinafter sometimes referred to as "CAR gene") and use thereof (expression cassette, vector, method for preparing a gene-modified lymphocyte expressing the CAR, gene-modified lymphocyte expressing the CAR, and use thereof). The CAR gene of the present invention encodes a CAR having the above structure. Therefore, by introducing it into a target cell in vitro and expressing it, a gene-modified lymphocyte (CAR gene-introduced lymphocyte) expressing the CAR of the present invention on the cell surface can be obtained. The CAR gene-introduced lymphocyte can be used for CAR therapy. A specific example of the sequence of the CAR gene is shown in SEQ ID NO: 3. The CAR gene has, from the 5'end toward the 3' end, a region (SEQ ID NO: 5) encoding the EphrinB2 extracellular domain (SEQ ID NO: 1), a linker sequence (SEQ ID NO: 6), a region (SEQ ID NO: 7) encoding CD28 (including transmembrane domain and intracellular domain), and a region (SEQ ID NO: 8) encoding the CD3ζ intracellular domain, which are arranged in series.

An expression cassette (hereinafter may be referred to as "CAR expression cassette") can be constructed by using a CAR gene. The CAR expression cassette includes a promoter and a CAR gene under control of the promoter. Usually, the CAR gene is disposed downstream of the promote so as to be under control of the promoter. Examples of the promoter used in the CAR expression cassette include CMV-IE (cytomegalovirus early gene-derived promoter), SV40ori, retrovirus LTP, SR*α*, EF1 *α*, and *β* actin promoter. The promoter is operably linked to the CAR gene. "The promoter is operably linked to the CAR gene." has the same meaning with "the CAR gene is disposed under control of the promoter", and usually, the CAR gene is linked to the 3' terminal side of the promoter directly or via other sequence. A poly-A additional signal sequence is disposed downstream of the CAR gene. Transcription is terminated by the use of the poly-A additional signal sequence. The poly-A additional signal sequence may be, for example, the poly-A additional sequence of SV40, or the poly-A additional sequence of a bovine-derived growth hormone gene.

The expression cassette may include, for example, a gene for detection (for example, a reporter gene, cell or tissue-specific gene, or selectable marker gene), an enhancer sequence, and a WRPE sequence. The gene for detection is used for the judgement of success/failure or efficiency of the introduction the expression cassette, detection of CAR gene expression or judgement of the expression efficiency, and selection and collection of the cells having expressed the CAR gene. On the other hand, the enhancer sequence is used for improving the expression efficiency. Examples of the gene for detection include the neo gene imparting resistance against neomycin, the npt gene (Herrera Estrella, EMBO J.2 (1983), 987-995) and npt II gene (Messing & Vierra.Gene 1 9: 259-268(1982)) imparting resistance against kanamycin, the hph gene imparting resistance against hygromycin (Blochinger & Diggl mann, Mol Cell Bio 4: 2929-2931), and the dhfr gene imparting resistance against Methotrexate (Bourouis et al.,EMBO J.2(7)) (the aforementioned are marker genes); genes of fluorescence proteins such as the luciferase gene (Giacomin, P1.Sci.116 (1996), 59 to 72; Scikantha, J.Bact.178 (1996), 121), the β-glucuronidase (GUS) gene, GFP (Gerdes, FEBS Lett.389 (1996), 44-47), and their variants (EGFP and d2EGFP) (the aforementioned are reporter genes); and the epidermal growth factor receptor (EGFR) gene deficient in the intracellular domain. The gene for detection is linked to the CAR gene through, for example, a bicistronic control sequence (for example, internal ribosome entry site (IRES)) and a sequence coding a self cleavage peptide. Examples of the self cleavage peptide include, but not limited to, the 2A peptide (T2A) derived from Thosea asigna virus. Known examples of the self cleavage peptide include the 2A peptide (F2A) derived from the Foo-and-mouse disease virus (FMDV), the 2A peptide (E2A) derived from equine rhinitis A virus (ERAV), and the 2A peptide (P2A) derived from porcine teschovirus (PTV-1).

The car expression cassette is included in a vector for its delivery to a target cell. The "vector" herein refers to a nucleic acid molecule capable of delivering a nucleic acid molecule inserted therein into a target (target cell). The form thereof, the origin thereof and the like are not particularly limited. A variety of the vectors can be employed. Examples of preferred vector include a viral vector. However, non-viral vector can be used. The viral vector cleverly uses the phenomenon of the infection of a virus to a cell, and provides a high gene introduction efficiency. As the viral vectors, for example, retrovirus vector, lentivirus vector, adenovirus vector, adeno-associated virus vector, herpesvirus vector, and Sendai virus vector have been developed. Among them, the retrovirus vector, lentivirus vector, and adeno-associated virus vector are expected to achieve stable and long-term expression, because the target genes included in these vectors are integrated in the host chromosomes. These viral vectors can be prepared according to known methods, or using a commercially available kit. Examples of the non-viral vector include plasmid vector, liposome vector, positively charged liposome vector (Felgner, P.L., Gadek, T.R., Holm, M.et al., Proc.Natl.Acad.Sci., 84: 7413-7417, 1987), YAC vector, and BAC vector.

The gene introduction is performed in vitro and is preferably carried out by a transposon method. The transposon method is one of the non-viral gene introduction methods. Transposon is the generic name of short gene sequences causing a gene transposition conserved during the process of evolution. A pair of a gene enzyme (transposase) and its specific recognition sequence causes gene transposition. The transposon method may be, for example, the piggyBac transposon method. The piggyBac transposon method uses the transposon isolated from insects (Fraser MJ et al., Insect Mol Biol.1996 May; 5(2): 141-51.; Wilson MH et al., Mol Ther.2007 Jan; 15(1): 139-45.), and allows highly efficient integration into mammal chromosomes. The piggyBac transposon method is actually used for the introduction of the CAR gene (for example, see Nakazawa Y, et al., J Immunother 32: 826-836, 2009; Nakazawa Y et al., J Immunother 6: 3-10, 2013) . The transposon method applicable to the present invention is not limited to that using piggyBac, and may use a method using transposon, for example, Sleeping Beauty (Ivics Z, Hackett PB, Plasterk RH, Izsvak Z (1997) Cell 91: 501-510.), Frog Prince (Miskey C, Izsvak Z, Plasterk RH, Ivics Z (2003) Nucleic Acids Res 31: 6873-6881.), Toll (Koga A, Inagaki H, Bessho Y, Hori H.Mol Gen Genet.1995 Dec 10; 249 (4): 400-5.; Koga A, Shimada A, Kuroki T, Hori H, Kusumi J, Kyono-Hamaguchi Y, Hamaguchi S.J Hum Genet. 2007; 52(7): 628-35.Epub 2007 Jun 7.), Tol2 (Koga A, Hori H, Sakaizumi M (2002) Mar Biotechnol 4: 6-11.; Johnson Ha mL et MR, Yergeau DA, Kuliyev E, Takeda M, Taira M, Kawakami K, Mead PE (2006) Genesis 44: 438-445.; Choo BG, Kondrichin I, Parinov S, Emelyanov A, Go W, Toh WC, and Korzh V (2006) BMC Dev Biol 6: 5.).

The introduction operation by the transposon method may be carried out by an ordinary method with reference to past literatures (for example, for the piggyBac transposon method, see Nakazawa Y, et al., J Immunother 32: 826-836, 2009, Nakazawa Y et al., J Immunother 6: 3-10, 2013, Saha S, Nakazawa Y, Huye LE, Doherty JE, Galvan DL, Rooney CM, Wilson MH. J Vis Exp. 2012 Nov 5; (69): e4235, Saito S, Nakazawa Y, Sueki A, et al. Anti-leukemic potency of piggyBac-mediated CD19-specific T cells against refractory Philadelphia chromosome-positive acute lymphoblastic leukemia. Cytotherapy. 2014; 16: 1257-69.).

In a preferred embodiment of the present invention, the piggyBac transposon method is used. Typically, in the piggyBac transposon method, a vector including the gene coding piggyBac transposase (transposase plasmid) and a vector having a structure wherein the desired nucleic acid construct (CAR expression cassette) is sandwiched between piggyBac inverted repeat sequences (transposon plasmid) are prepared, and these vectors are introduced (transfected) to the target cell. The transfection may use various methods such as electroporation, nucleofection, lipofection, or calcium phosphate method.

Examples of a target cell (the cell into which the CAR gene is introduced) include CD4-positive CD8-negative T-cells, CD4-negative CD8-positive T-cells, T-cells prepared from iPS cells, αβ-T-cells, γδ-T-cells, NK cells, and NKT cells. Various cell populations may be used, as long as they contain the above-described lymphocytes or precursor cells. PBMCs (peripheral blood mononuclear cells) collected from the peripheral blood is one of the preferred target cells. More specifically, in a preferred embodiment, gene introduction operation is carried out on the PBMCs. The PBMCs may be prepared by an ordinary method. The method for preparing the PBMCs may refer to, for example, Saha S, Nakazawa Y, Huye LE, Doherty JE, Galvan DL, Rooney CM, Wilson MH. J Vis Exp.2012 Nov 5; (69): e4235. Unless otherwise specified, the cells (for example, T-cells) herein are human cells.

The CAR gene-introduced lymphocytes obtained by the above steps are typically subjected to activation treatment. For example, the CAR gene-introduced lymphocytes are activated by stimulation with an anti-CD3 antibody and an anti-CD28 antibody. This treatment also usually promotes survival and proliferation of the CAR gene-introduced lymphocytes. For example, stimulation by the anti-CD3 antibody and anti-CD28 antibody can be applied by culturing in a culture vessel (for example, culture dish) coated with the anti-CD3 antibody and anti-CD28 antibody on the culture surface for 1 to 20 days, preferably 3 to 14 days, and more preferably 5 to 10 days. The anti-CD3 antibody (for example, CD3pure antibody provided by Miltenyi Biotec) and the anti-CD28 antibody (for example, CD28pure antibody provided by Miltenyi Biotec) are readily and commercially available. Magnetic beads (for example, Dynabeads T-Activator CD3/CD28 provided by VERITAS) coated with the anti-CD3 antibody and anti-CD28 antibody may be used to apply the stimulation. The anti-CD3 antibody is preferably "OKT3" clone. In order to promote recovery from injury/disturbance by gene introduction operation, the activation treatment is preferably carried out about 8 to 48 hours (preferably 16 to 24 hours) after the gene introduction operation, rather than immediately after the gene introduction operation.

In order to improve the survival rate/proliferation rate of the cells, it is preferred to use a culture solution containing a T-cell growth factor in the activation treatment. The T-cell growth factor is preferably IL-15. Preferably, a culture solution containing IL-15 and IL-7 is used. The concentration of IL-15 is, for example, from 1 ng/mL to 20 ng/mL, and preferably from 5 ng/mL to 10 ng/mL. The concentration of IL-7 is, for example, from 1 ng/ mL to 20 ng/ mL, and preferably from 5 ng/mL to 10 ng/mL. The T-cell growth factor such as IL-15 or IL-7 may be prepared according to a common procedure. Alternatively, a commercial product may be used. Although the use of animal T-cell growth factors other than human ones will not be excluded, the T-cell growth factor used herein is usually derived from human (may be a recombinant). The growth factors such as human IL-15 and human IL-7 are readily available (for example, provided by Miltenyi Biotec, R&D systems).

A medium containing blood serum (for example, human blood serum or fetal bovine serum) may be used, but the use of a serum-free medium allows the preparation of cells having advantages of high safety in clinical application, and safe advantages of a high level of safety and little difference in the culture efficiency among blood serum lots. Specific example of the serum-free medium for lymphocytes include TexMACS^{™} (Miltenyi Biotec) and AIM V (registered trademark) (Thermo Fisher Scientific). When a blood serum is used, the blood serum is preferably an autologous serum, or a blood serum collected from a patient to receive administration of CAR gene-introduced lymphocytes obtained by the preparation method of the present invention. The basal culture medium is the one suitable for culture of lymphocytes, and a specific example is the above-listed TexMACS^{™} AIM V (registered trademark). Other culture conditions may be common ones, as long as they are suitable for the survival and proliferation of lymphocytes. For example, the lymphocytes are cultured in a CO2 incubator adjusted at 37°C (CO2 concentration: 5%).

After activating treatment, the cells are collected. The collecting operation may follow an ordinary method. For example, the cells are collected by pipetting or centrifugation. In one preferred embodiment, before the collecting operation, the cells after activating treatment is cultured in the presence of a T-cell growth factor. This step allows efficient expanded culture, and increases the cell survival rate. The T-cell growth factor used herein may be, for example, IL-15 or IL-7. In the same manner as in the activating treatment, the cells may be cultured in a medium containing IL-15 and IL-7. The culture period is for example from 1 to 21 days, preferably from 5 to 18 days, and more preferably from 10 to 14 days. If the culture period is too short, the number of cells will not sufficiently increase, and if the culture period is too long, cell activity (survival ability) may decrease, and the cell may cause exhaustion/fatigue or the like. The cells may be subcultured during the culture. During the culture, the medium is replaced as necessary. For example, about 1/3 to 2/3 the culture solution is replaced with a new medium once in three days.

In one embodiment of the present invention, as a CAR gene-introduced lymphocyte, chimeric antigen receptor gene-modified T cells which acquired virus specificity (referred to as "virus specificity-acquired CAR-T cells") are prepared. The virus specificity-acquired CAR-T cells have important advantages in clinical application, such as their use in autotransplantation improves internal persistence by stimulation from a viral T cell receptor, and their use in allogeneic transplantation further allows the preparation of CAR-T from a transplanted donor owing to the reduction of allogeneic immunity reaction (GVHD), and creates possibility of drug formulation of CAR-T cells from a third party donor. Actually, there is a report that virus specificity-acquired CAR-T cells survive longer in the body (Pule MA, et al. Nat Med. 2008 Nov; 14 (11): 1264-70.). In addition, the report of a third party-derived EBV-specific CTL clinical study (Annual Review Blood 2015, published in January 2015, Chugai-Igakusha) supports high level of safety of virus specificity-acquired cytotoxic T cells (CTLs).

The preparation method of this embodiment includes the following steps (i) to (iv).
(i) A step of preparing non-proliferative cells holding a viral peptide antigen, which are obtained by stimulating a group of cells including T cells using an anti-CD3 antibody and an anti-CD28 antibody followed by culturing in the presence of the viral peptide antigen and a treatment for causing the cells to lose their proliferation capability
(ii) A step of obtaining gene-modified T cells by introducing a EPHB4 receptor-specific chimeric antigen receptor gene into target cells in vitro
(iii) A step of mixing the non-proliferative cells prepared by step (i) with the gene-modified T cells obtained by step (ii), and co-culturing the mixed cells
(iv) A step of collecting the cells after culture.

In step (i), firstly, the group of cells including T cells are stimulated with an anti-CD3 antibody and an anti-CD28 antibody, thereby obtaining activated T cells. As the "a group of cells including T cells", preferably, PBMCs (peripheral blood mononuclear cells) collected from the peripheral blood are used. The "group of cells including T cells" herein may be, for example, the PBMCs which have been purified to increase the T-cell content, or mononuclear cells collected from the peripheral blood by pheresis.

For example, the T cells in a group of cells can be stimulated with an anti-CD3 antibody and an anti-CD28 antibody by culturing them in a culture vessel (for example, culture dish) coated with an anti-CD3 antibody and an anti-CD28 antibody on the culturing surface for three hours to three days, preferably six hours to two days, and more preferably from 12 hours to one day. The anti-CD3 antibody (for example, the trade name CD3pure antibody provided by Miltenyi Biotec may be used) and the anti-CD28 antibody (for example, the trade name CD28pure antibody provided by Miltenyi Biotec may be used) are commercially available and are easily available. The stimulation in step (i) may be carried out using the magnetic beads coated with an anti-CD3 antibody and an anti-CD28 antibody (for example, Dynabeads T-Activator CD3/CD28 provided by VERITAS). The anti-CD3 antibody is preferably "OKT3" clone.

After obtaining the activated T cells, they are subjected to culturing in the presence of the viral peptide antigen and a treatment for causing the cells to lose their proliferation capability. As a result of this, non-proliferative "activated T cells holding a viral peptide antigen on the cell surface" (hereinafter referred to as "viral peptide-holding non-proliferative cells") are obtained. The order of culturing in the presence of the viral peptide antigen and a treatment for causing the cells to lose their proliferation capability is not particularly limited. Accordingly, the proliferation capability may be lost after culturing in the presence of the viral peptide antigen, or the cells may be cultured in the presence of the viral peptide antigen after they were caused to lose their proliferation capability. Preferably, the former order is adopted in the expectation that the intake of the viral peptide antigen would be better than before the loss of proliferation capability.

The "treatment for losing the proliferation capability" is typically irradiation, but may use a drug. The irradiation is carried out by, for example, using a γ-ray, at an intensity of 25 Gy to 50 Gy, for 15 to 30 minutes.

In order to culture the cells in the presence of the viral peptide antigen, for example, a culture medium containing the viral peptide antigen is used. Alternatively, the viral peptide antigen may be added to the culture medium during culturing. The addition concentration of the viral peptide antigen is, for example, from 0.5 µg/ mL to 1 µg/mL. The culture period is, for example, from 10 minutes to 5 hours, and preferably from 20 minutes to 3 hours. The "viral peptide antigen" in the present description means an epitope peptide or a long peptide containing an epitope which can induce cytotoxic T cells (CTLs) specific to a specific virus. Examples of the viral peptide antigen include, but not limited to, antigen peptides of adenovirus (AdV) (for example, see WO 2007015540 A1), antigen peptides of cytomegalovirus (CMV) (for example, see Japanese Unexamined Patent Application Publication No. 2002-255997, Japanese Unexamined Patent Application Publication No. 2004-242599, and Japanese Unexamined Patent Application Publication No. 2012-87126), and antigen peptides of Epstein-Barr virus (EBV) (for example, see WO 2007049737 A1, Japanese Patent Application No. 2011-177487, and Japanese Unexamined Patent Application Publication No. 2006-188513). The viral peptide antigen can be prepared by a common procedure (for example, a solution-phase synthesis method or a solid-phase synthesis method) based on the sequence information. Some viral peptide antigens are commercially available (for example, provided by Medical & Biological Laboratories Co., Ltd., Takara Bio, Inc., and Miltenyi Biotec).

One antigen peptide may be used, but usually two or more antigen peptides (an antigen peptide mixture) are used. For example, an AdV antigen peptide mixture, a CMV antigen peptide mixture, or an EBV antigen peptide mixture, or a combination of two or more of these antigen peptide mixtures (for example, a mixture of the AdV antigen peptide mixture, CMV antigen peptide mixture, and EBV antigen peptide mixture) is used. The combination of two or more antigen peptides allows obtainment of plural activated T cells having different targets (antigen peptides), which would increase the subjects (patients) to whom the CAR-T cells obtained by the preparation method of the present invention is effective (the improvement of cover rate). When determining which virus the antigen peptide to be used is derived from, the use of the CAR-T cells obtained by the preparation method of the present invention, specifically the disease and the disease state of the patient to be treated may be considered. The AdV antigen peptide mixture, CMV antigen peptide mixture, and EBV antigen peptide mixture are commercially available (for example, PepTivator (registered trademark) AdV5 Hexon, PepTivator (registered trademark) CMV pp65, PepTivator (registered trademark) EBV EBNA-1, PepTivator (registered trademark), and EBV BZLF1 provided by Miltenyi Biotec, and PepMix^{™} Collection HCMV, PepMix^{™} EBV (EBNA1) provided JPT Peptide Technologies, and the like) are easily available.

Step (ii) corresponds to the gene introduction operation (introduction of the CAR gene) explained above, a variety of gene introduction methods can be employed. Preferably, a transposon method is used. Through this step, the gene-modified T cells (CAR-T cells) are obtained.

In step (iii), the non-proliferative cells (viral peptide-holding non-proliferative cells) prepared in step (i) and the gene-modified T cells obtained in step (ii) are mixed, and the mixed cells are co-cultured. As a result of this, stimulation through the costimulatory molecules by the non-proliferative cells and stimulation by the viral antigen peptide are added, whereby the virus antigen specific gene-modified T cells are activated, and their survival and proliferation are promoted.

The ratio between the number of the non-proliferative cells used for co-culturing and the number of the gene-modified T cells (the number of non-proliferative cells/the number of gene-modified T cells) is not particularly limited, and, for example, from 0.025 to 0.5.

In this step, in principle, stimulation by an anti-CD3 antibody and an anti-CD28 antibody is not applied for the purposes of, for example, selectively proliferating the virus specificity-acquired CAR-T cells, or preventing exhaustion and fatigue of the T cells by avoiding strong stimulation. On the other hand, in order to increase the survival rate/proliferation rate of the cells, it is preferred that a culture solution containing a T-cell growth factor be used during the co-culturing. The T-cell growth factor is preferably IL-15. Preferably, a culture solution containing IL-15 and IL-7 are used. The addition amount of IL-15 is, for example, from 5 ng/mL to 10 ng/mL. In the same manner, the addition amount of IL-7 is, for example, from 5 ng/mL to 10 ng/mL. The T-cell growth factor such as IL-15 or IL-7 may be prepared according to a common procedure. Alternatively, a commercial product may be used. Although the use of animal T-cell growth factors other than human ones will not be excluded, the T-cell growth factor used herein is usually derived from human (may be a recombinant). The growth factors such as human IL-15 and human IL-7 are readily available (for example, provided by Miltenyi Biotec, R&D systems).

A culture medium containing blood serum (for example, human blood serum or fetal bovine serum) may be used, but the use of a serum-free medium allows the preparation of cells having advantages of high safety in clinical application, and little difference in the culture efficiency among blood serum lots. Specific examples of the serum-free medium for T cells include TexMACS^{™} (Miltenyi Biotec) and AIM V (registered trademark) (Thermo Fisher Scientific). When a blood serum is used, the blood serum is preferably a self-blood serum, or a blood serum collected from the individual who is the origin of the gene-modified T cells obtained in step (2) (typically, the patient to receive administration of the chimeric antigen receptor gene-modified T cells obtained by the preparation method of the present invention). The basal culture medium is the one suitable for culture of T cells, and specific examples include the above-listed TexMACS^{™} and AIM V (registered trademark). Other culture conditions may be common ones, as long as they are suitable for the survival and proliferation of T cells. For example, the culture is carried out in a CO₂ incubator adjusted at 37°C (CO₂ concentration: 5%).

The viral peptide-holding non-proliferative cells may be added during step (iii). Alternatively, the co-cultured cells are collected, mixed with the viral peptide-holding non-proliferative cells, and then co-culturing is carried out again. These operations may be repeated twice or more times. In this manner, the improvement of the induction rate of the virus specificity-acquired CAR-T cells and the increase of the number of the virus specificity-acquired CAR-T cells are expected by carrying out plural times of the stimulation or activation using the viral peptide-holding non-proliferative cells. The viral peptide-holding non-proliferative cells used herein are prepared anew, or a portion of the preserved cells which have been prepared in step (i).

In step (iii), the period of co-culturing is, for example, from one day to 21 days, preferably from five days to 18 days, and more preferably from 10 days to 14 days. If the culture period is too short, sufficient effect cannot be obtained, and if the culture period is too long, the activity (vital force) of the cells may decrease, and the cells may be exhausted or fatigued.

Before the co-culturing with the viral peptide-holding non-proliferative cells, the gene-modified T cells obtained in step (ii) may be co-cultured with viral peptide-holding non-proliferative PBMCs (peripheral blood mononuclear cells). In this embodiment, the cells obtained by co-culturing the gene-modified T cells obtained in step (ii) with the viral peptide-holding non-proliferative PBMCs, and the viral peptide-holding non-proliferative cells prepared in step (i) are mixed, and the mixture is co-cultured. The viral peptide-holding non-proliferative PBMCs herein can be prepared by subjecting PBMCs to culturing in the presence of a viral peptide antigen and a treatment for causing them to lose their proliferation capability. Specifically, for example, PBMC isolated from the peripheral blood are irradiated, and then cultured in the presence of a viral peptide antigen, thus obtaining viral peptide-holding non-proliferative PBMCs. The number of blood collection for carrying out the present invention can be reduced by preparing viral peptide-holding non-proliferative PBMCs using a portion of the PBMCs isolated from the peripheral blood obtained by one time of blood collection, and preparing gene-modified T cells from another portion, which will bring a markedly big advantage in clinical application. In particular, when the viral peptide-holding non-proliferative cells (the cells used for the second step co-culturing) are prepared by carrying out step (i) using the remaining PBMCs, the three kinds of necessary cells, more specifically, the gene-modified T cells, viral peptide-holding non-proliferative PBMCs used for co-culturing with these cells, and the viral peptide-holding non-proliferative cells used for the second step co-culturing can be prepared by one time of blood collection, which markedly reduces the burden imposed on the patient in the treatment using the CAR-T cells obtained in the present invention.

In step (iv) following step (iii), the cells after culture are collected. The collection operation may use an ordinary method. For example, the collection is carried out by pipetting or centrifugation treatment. The step of culturing the co-cultured cells in the presence of the T-cell growth factor (expanded culturing) may be carried out between step (iii) and step (iv). For this cell expansion, viral peptide-holding non-proliferative cells may be added, or viral peptide-holding non-proliferative cells may be added during the cell expansion.

In another embodiment, the gene-modified T cells obtained by the same operation as in step (ii) are co-cultured with viral peptide-holding non-proliferative PBMCs (peripheral blood mononuclear cells), and then stimulated with an anti-CD28 antibody (an anti-CD3 antibody may be used in combination). Subsequently, after culturing (for example, expanded culturing) as necessary, the cells are recovered to obtain CAR-T cells (virus specificity-acquired CAR-T cells as the CAR gene-introduced lymphocytes of the present invention).

### 3. CAR gene-introduced lymphocytes and uses thereof

The further aspect of the present invention relates to the gene-modified lymphocyte expressing chimeric antigen receptors obtained in the preparation method of the present invention (hereinafter referred to as "CAR gene-introduced lymphocytes of the present invention") and uses thereof. The CAR gene-introduced lymphocytes of the present invention can be used in a method of treatment of tumor/cancer (hereinafter referred to as "target diseases") in which EPHB4 receptors are highly expressed. The target disease is selected from the group consisting of rhabdomyosarcoma, lung cancer, bowel cancer, malignant mesothelioma, esophagus cancer, breast cancer, ovarian cancer, and melanoma, head and neck cancer. "Treatment" include alleviation (moderation) of symptoms or associated symptoms characteristic to the target diseases, inhibition or retard of deterioration of symptoms .

The CAR gene-introduced lymphocytes of the present invention may be prepared in the form of a cell preparation. The cell preparation of the present invention contains the CAR gene-introduced lymphocytes of the present invention in a therapeutically effective amount. For example, 1 × 104 to 1 × 1010 cells are contained for one administration (one dose). The cell preparation may contain dimethylsulfoxide (DMSO) or serum albumin for the purpose of cell protection, antibiotics for the purpose of preventing bacterial contamination, and various components for (for example, vitamins, cytokine, growth factors, and steroids) for the purpose of cell activation, proliferation, or inductive differentiation.

The administration route of the CAR gene-introduced lymphocytes or cell preparation of the present invention is not particularly limited. For example, they are administered by intravenous injection, intraarterial injection, intraportal injection, intradermal injection, hypodermic injection, intramuscular injection, or intraperitoneal injection. Local administration may be used in place of systemic administration. Examples of the local administration include direct injection into the target tissues, body parts, and organs. The administration schedule may be made according to the sex, age, body weight, and pathology of the subject (patient). A single dose or continuous or periodical multiple doses may be used.

In the use ofg the CAR gene-introduced lymphocytes of the present invention in a method of treatment, a therapeutically effective amount of the CAR gene-introduced lymphocytes is administered to a patient. The CAR gene-introduced lymphocytes of the present invention exhibit the characteristic of exerting an antitumor effect on tumors that express EPHB4 protein on the cell membrane surface by their characteristic construction. Therefore, they can be used to treat specific tumor groups, i.e., rhabdomyosarcoma, lung cancer, bowel cancer, malignant mesothelioma, esophagus cancer, breast cancer, ovarian cancer, melanoma, and head and neck cancer.

### 4. Vector and kit for preparing CAR gene-introduced lymphocytes

Another aspect of the present invention relate to a vector (CAR gene-introduced lymphocyte preparation vector) and a kit (CAR gene-introduced lymphocyte preparation kit) usable in the preparation method of the present invention. The CAR gene-introduced lymphocyte preparation vector of the present invention includes a CAR expression cassette, and allows the introduction of the expression cassettes into the target cell. The CAR expression cassette includes the CAR gene, a promoter necessary for the expression of the CAR gene (for example, CMV-IE, SV40ori, retrovirus LTP, SRα, EF1α, or β actin promoter). The vector of the present invention may include a gene for detection (for example, reporter gene, cell or tissue-specific gene, or selectable marker gene), an enhancer sequence, and a WRPE sequence.

Preferably, the vector of the present invention is constructed as a vector used in the transposon method. In this case, typically, the vector has a structure wherein a CAR expression cassette is sandwiched between a pair of transposon inverted repeat sequences (for example, they are disposed in this order: 5' end transposon inverted repeat sequence, CAR expression cassette, and 3' end transposon inverted repeat sequence).

One embodiment of the kit of the present invention is suitable to the method for preparing CAR gene-introduced lymphocytes using the transposon method. The kit contains the CAR vector including the CAR expression cassette sandwiched between a pair of transposon inverted repeat sequences, and a transposase expression vector. The transposase is selected so as to correspond to the pair of transposon inverted repeat sequences integrated into the CAR vector. For example, a combination of a piggyBac inverted repeat sequence and piggyBac transposase is used.

The kit of the present invention may include the reagent, instrument, or apparatus used for the gene introduction operation, and the reagent, instrument, or apparatus used for the detection and selection of the transformant. An instruction manual is usually attached to the kit of the present invention.

### [Examples]

The following study was made to further advance the clinical application of CAR therapy.

### <Cytocidal effect of EPHB4-CAR-T cells>

1. Material and method
   (1) Preparation of pIRII-EPHB4-CAR vector
      (I) The previously-reported CD19.CAR expression piggyBac transposon vector (pIRII-CAR.CD19) (Saito S, Nakazawa Y, Sueki A, Matsuda K, Tanaka M, Yanagisawa R, Maeda Y, Sato Y, Okabe S, Inukai T, Sugita K, Wilson MH, Rooney CM, Koike K. Anti-leukemic potency of piggyBac-mediated CD19-specific T cells against refractory Philadelphia chromosome-positive acute lymphoblastic leukemia. Cytotherapy. 2014; 16; 1257-69) was cleaved with both restriction enzymes XhoI and BbvCI (New England Biolab, Ipswich, MA, USA).
      (II) mRNA was extracted from human neuroblastoma cell line SH-SY5Y, which is known to highly express EFNB2 genes, and cDNA was synthesized. EFNB2 is a membrane-bound protein having a sequence of 333 amino acids (SEQ ID NO: 2), and its extracellular domain (SEQ ID NO: 1) is known to include amino acids up to the 227th amino acid from the N-terminus. A PCR primer capable of specifically amplifying this site (XhoI-EFNB2 forward primer: ATCTCGAGATGGCTGTGAGAAGGG (SEQ ID NO: 9) and an EFNB2 ECD-BbvCI reverse primer: ATCCTCAGCATAAGGCCACTTCGGAAC (SEQ ID NO: 10)) was prepared, and the previously obtained cDNA was used as a template to perform a PCR reaction. In this PCR primer sequence, an XhoI restriction enzyme recognition site was inserted in the Forward primer, and a BbvCI recognition site was inserted in the Reverse primer, in advance.
      (III) The 699 bp DNA fragment obtained in (II) was cloned into a pCR-Blunt plasmid using ZeroBlunt PCR Cloning Kit (Thermo Fisher Scientific, Carlsbad, CA, USA). Using this plasmid, *E. coli* was transformed, mass-amplified, and extracted. The pCR-Blunt plasmid into which the extracted EFNB2 extracellular domain base sequence was inserted was cleaved with both restriction enzymes XhoI and BbvCI.
      (IV) The 4971 bp DNA fragment obtained in (I) and the 689 bp DNA fragment obtained in (III) were ligated using DNA Ligation kit (Takarabio, Otsu, Shiga, Japan).
      (V) Competent cells were used to mass-amplify the 5660 bp circular DNA plasmid (SEQ ID NO: 4) obtained in (IV).
      (VI) The entire base sequence was confirmed using Applied Biosystems 3130 Genetic Analyzer (Applied Biosystems, Waltham, MA, USA) (see the Sequence Listing).
   (2) Preparation of EPHB4-CAR-T cells
      (I) Mononuclear cells (PBMC) were separated from about 10 ml of peripheral blood using specific gravity centrifugation.
      (II) The pIRII-EPHB4-CAR vector (5 µg) and the pCMV piggyBac vector (5 µg) were gene-introduced into 1×10 ⁷ PBMCs using electroporation (Program EO-115) by combination of 4D-Nucleofector^{™} device and P3 Primary Cell 4D-Nucleofector^{™}X kit (Lonza, Basel, Switzerland).
      (III) 1x10⁶ irradiated PBMCs pulsed with four viral antigen peptides (MACS GMP PepTivator; AdV5 Hexon, CMV pp65, EBV EBNA-1, EBV BZLF1, Miltenyi Biotec, Auburn, CA) were mixed with the gene-introduced cells obtained in (II). The mixtures were placed in one well of a 24-well culture plate which was filled with 2 ml of TexMACS medium (Miltenyi) added with interleukin (IL)-7 (10 ng/ml, Miltenyi) and IL-15 (5 ng/ml, Miltenyi) and also which was immobilized with an anti-CD28 antibody (Miltenyi). Three days after gene introduction, gene-introduced cells were transferred to one well of a non-immobilized 24-well culture plate. At that time, 1 ml of the IL-7/IL-15-added TexMACS medium was exchanged. Seven days after gene introduction, the gene-introduced cells were transferred to a G-Rex 10 incubator (Wilson Wolf Manufacturing Inc, New Brighton, MN) filled with 30 ml of a TexMACS medium added with IL-7/IL-15 (5 ng/ml). The cells were recovered on Day 14 after gene introduction. The expression of CAR protein was confirmed by flow cytometry using part of the cells. The thus-prepared CAR-T cells are referred to as EPHB4-CAR-T cells. As a control group, CD19-CAR-T cells in which the previously reported pIRII-CAR.CD19 vector was gene-introduced were also amplified and cultured in the same manner.
   (3) Co-culture experiment 1

As a highly EPHB4-expressing tumor, a tumor cell line Rh30 (in which EPHB4 was highly expressed and CD19 was low expressed) of rhabdomyosarcoma which is one of the typical childhood cancers was used. Human B cell lymphoma Raji cells (in which EPHB4 was low expressed and CD19 was highly expressed) were used as a control low EPHB4-expressing tumor. Into one well of a 24-well culture plate, 2x10⁵ CAR-T cells (CD19-CAR-T cells or EPHB4-CAR-T cells) and 1x10⁵ tumor cells (Rh30 cells or Raji cells) were inserted (a ratio of CAR-T cells to tumor cells: 2:1), and co-cultured in 1 ml of a 10% fetal bovine serum-containing DMEM medium for 3 days. Three wells were prepared for each combination.

At 0 hours and 72 hours after the start of co-culture, the cells were recovered for each well, and stained with an anti-CD3-APC antibody and an anti-IGF1R-PE antibody. Then, the ratio of CD3-positive cells (T cells) to IGF1R-positive cells (Rh30 cells) was measured by flow cytometry. The control Raji cells were stained with the anti-CD3-APC antibody and the anti-CD19-FITC antibody, and then the ratio of CD3-positive cells (T cells) to CD19-positive cells (Raji cells) was measured by flow cytometry.

### (4) Co-culture experiment 2

CD19-CAR-T cells or EPHB4-CAR-T cells and Rh30 cells were co-cultured in a cell ratio of 2:1, and ELISA was performed to quantify IFNγ released into the culture supernatant on Day 0 and Day 3 of co-culture.

### 2. Results

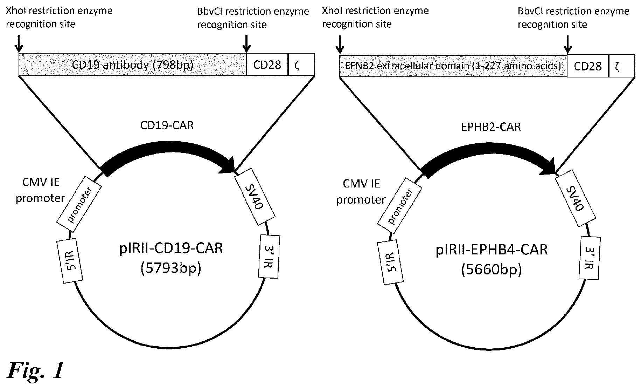

The construction of the EPHB4-CAR expression vector (pIRII-EPHB4-CAR) in which the EFNB2 extracellular domain was inserted as the antigen recognition site is shown in FIG. 1. In addition, the nucleotide sequence (SEQ ID NO: 4) of the EPHB4-CAR expression vector is shown in the Sequence Listing. The sequence of SEQ ID NO: 4 includes a region (6 bp to 687 bp, SEQ ID NO: 5) encoding the EphrinB2 extracellular domain, a linker sequence (SEQ ID NO: 6), a region (702 bp to 1022 bp, SEQ ID NO: 7) encoding CD28 (including the transmembrane domain and the intracellular domain), and a region encoding the CD3ζ intracellular domain chain (1023 bp to 1361 bp, SEQ ID NO: 8).

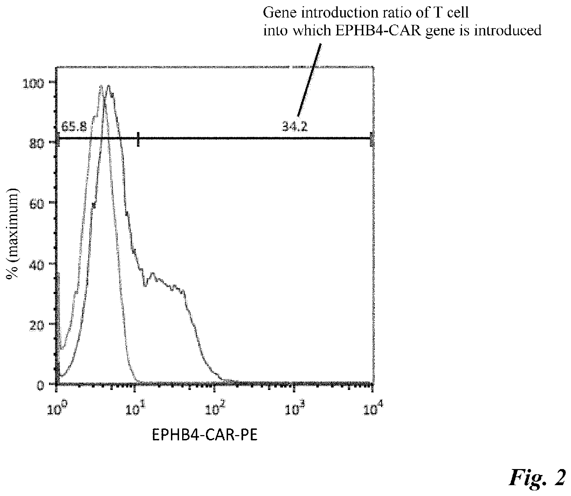

As a result of examining the CAR expression rate on the T cells on Day 15 after gene introduction, it was 34.2% (FIG. 2) for the T cells into which the EPHB4-CAR gene was introduced, and 55.1% for the T cells into which the CD19-CAR gene was introduced.

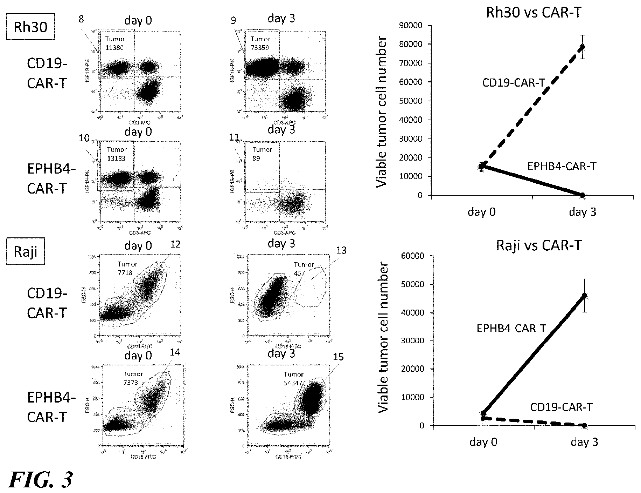

The results of co-culture experiment 1 are shown in FIG. 3 and the following table. The EPHB4-CAR-T cells exhibited a high proliferation inhibitory effect specifically for the tumor (Rh30) in which EPHB4 was highly expressed.

**[Table 1]**

| | Rh30 (Rhabdomyosarcoma cell) | | Raji (B-cell lymphoma cells) | |
|---|---|---|---|---|
| | Day 0 | Day 3 | Day 0 | Day 3 |
| EPHB4-CAR-T cell | 15622±1745 | 77±14 | 4440±259 | 46064±5862 |
| CD19-CAR-T cell | 14796±576 | 78571±6279 | 2633±224 | 41±9 |

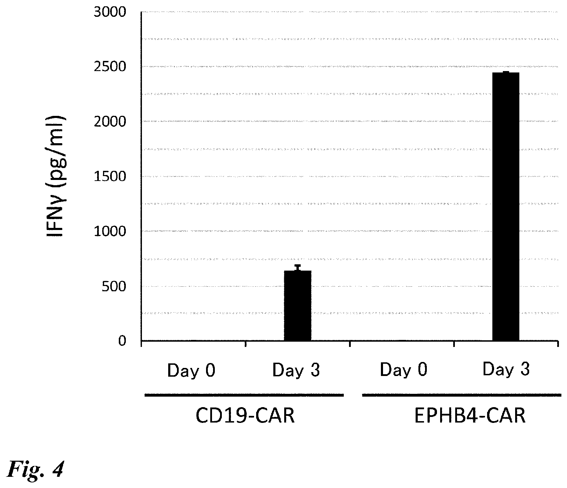

The results of co-culture experiment 2 are shown in FIG. 4. As corresponding to the results of co-culture experiment 1, the EPHB4-CAR-T cells were activated on Day 3 of co-culture and highly produced IFNγ.

### 3. Discussion

The EPHB4-CAR-T cells selectively and potently induced cell death to rhabdomyosarcoma cells in which EPHB4 was highly expressed. That is, it has been proved that the EPHB4-CAR-T cell developed this time can specifically kill a tumor in which EPHB4 is highly expressed. Therefore, treatment with the EPHB4-CAR-T cells (CAR-T therapy) is promising as a novel method for treating rhabdomyosarcoma. The EPHB4-CAR-T cells are greatly expected to be applied to the treatment of various tumors/cancers in which EPHB4 is highly expressed, such as rhabdomyosarcoma, lung cancer, bowel cancer, malignant mesothelioma, esophagus cancer, breast cancer, ovarian cancer, melanoma, and head and neck cancer.

### <Animal experiment>

The cytocidal effect of the EPHB4-CAR-T cells was verified in a mouse model. Tumor-carrying mice were prepared by subcutaneously inoculating immunodeficient mice (SCID Beige mice) with 2×10⁶ rhabdomyosarcoma cell lines Rh30 labeled with firefly luciferase.

One week after inoculation, 10×10⁶ CAR-T cells (CD19-CAR-T cells or EPHB4-CAR-T cells) were intravenously injected from the tail vein. The tumor size was then assessed every 7 days using the IVIS live imaging system (Sumisho Pharma International Co., Ltd.).

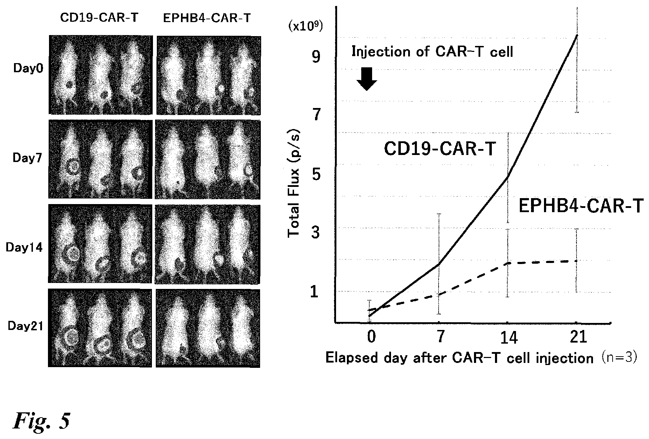

As shown in FIG. 5, a marked increase in the tumor was observed in the CD19-CAR-T cell administration group, while the EPHB4-CAR-T administration group showed a tumor growth suppressive effect.

### [Industrial Applicability]

Cell therapy (CAR therapy) using gene-modified lymphocytes (T cells, NK cells, etc.) into which a chimeric antigen receptor (CAR) gene or a T cell receptor (TCR) gene is introduced is expected as an effective treatment method for refractory cancer. The CAR gene-introduced lymphocytes provided by the present invention are specific to highly EPHB4 receptor-expressing cells and are, for example, promising therapeutic agents (cell preparations) for rhabdomyosarcoma. That is, the CAR gene-introduced lymphocytes of the present invention can be expected to induce cell death selectively and potently to rhabdomyosarcoma cells in which EPHB4 is highly expressed. Rhabdomyosarcoma is a typical refractory childhood cancer, and the prognosis for advanced rhabdomyosarcoma is extremely poor, and the development of new treatment is highly desired. The present invention meets such needs, and the significance thereof is extremely large.

### [Sequence Listing Free Text]

SEQ ID NO: 3: Explanation of artificial sequences: EFNB2-CAR gene
SEQ ID NO: 4: Explanation of artificial sequences: pIRII-EFNB2-CAR vector
SEQ ID NO: 6: Explanation of artificial sequence: linker
SEQ ID NO: 9: Explanation of artificial sequences: XhoI-EFNB2 forward primer
SEQ ID NO: 10: Explanation of artificial sequences: EFNB2 ECD-BbvCI reverse primer

[Sequence Listing]

## Claims

1. An EPHB4 receptor-specific chimeric antigen receptor comprising an extracellular domain including an EphrinB2 extracellular domain, a transmembrane domain, and an intracellular signal domain for an effector function of immunocytes.

2. The EPHB4 receptor-specific chimeric antigen receptor according to claim 1, wherein the EphrinB2 extracellular domain comprises an amino acid sequence of SEQ ID NO: 1.

3. The EPHB4 receptor-specific chimeric antigen receptor according to claim 1 or 2, wherein the intracellular signal domain includes an intracellular domain of a costimulatory molecule and CD3ζ.

4. The EPHB4 receptor-specific chimeric antigen receptor according to claim 3, wherein the costimulatory molecule is CD28.

5. The EPHB4 receptor-specific chimeric antigen receptor according to any one of claims 1 to 4, comprising a spacer domain between the extracellular domain and the transmembrane domain.

6. A gene encoding the EPHB4 receptor-specific chimeric antigen receptor according to any one of claims 1 to 5.

7. A method for preparing a gene-modified lymphocyte expressing a chimeric antigen receptor, comprising a step of introducing the gene according to claim 6 into a target cell in vitro, preferably wherein the introduction of the gene is carried out by a transposon method, and the target cell is a T cell.

8. The preparation method according to claim 7, wherein the transposon method is a piggyBac transposon method.

9. A gene-modified lymphocyte expressing the EPHB4 receptor-specific chimeric antigen receptor according to any one of claims 1 to 5, preferably wherein the lymphocyte is a T cell.

10. A cell preparation comprising a therapeutically effective amount of the gene-modified lymphocyte according to claim 9.

11. The cell preparation according to claim 10 or the gene-modified lymphocyte according to claim 9 for use in a method of treating a tumor or cancer selected from the group consisting of rhabdomyosarcoma, lung cancer, bowel cancer, malignant mesothelioma, esophagus cancer, breast cancer, ovarian cancer, melanoma, and head and neck cancer.

12. An expression cassette comprising a promoter and the gene according to claim 6 under control of the promoter.

13. A vector having the expression cassette according to claim 12, preferably wherein the vector has a structure that the expression cassette is sandwiched between a pair of transposon inverted repeat sequences.

14. A kit for preparing a gene-modified lymphocyte expressing an EPHB4 receptor-specific chimeric antigen receptor, the kit comprising the vector according to claim 13 and a transposase expression vector, preferably wherein the transposase is piggyBac transposase.

15. The cell preparation according to claim 10 or for use according to claim 11, which exerts a therapeutic effect by a cell death induction effect by EphrinB2-EPHB4 binding and a cytocidal effect via a chimeric antigen receptor.

## Patentansprüche

1. EPHB4-Rezeptor-spezifischer chimärer Antigenrezeptor, der eine extrazelluläre Domäne, einschließlich einer EphrinB2 extrazellulären Domäne, eine Transmembrandomäne und eine intrazelluläre Signaldomäne für eine Effektorfunktion von Immunzellen umfasst.

2. EPHB4-Rezeptor-spezifischer chimärer Antigenrezeptor gemäß Anspruch 1, wobei die EphrinB2 extrazelluläre Domäne eine Aminosäuresequenz von SEQ ID NO: 1 umfasst.

3. EPHB4-Rezeptor-spezifischer chimärer Antigenrezeptor gemäß Anspruch 1 oder 2, wobei die intrazelluläre Signaldomäne eine intrazelluläre Domäne eines kostimulatorischen Moleküls und CD3ζ umfasst.

4. EPHB4-Rezeptor-spezifischer chimärer Antigenrezeptor gemäß Anspruch 3, wobei das kostimulatorische Molekül CD28 ist.

5. EPHB4-Rezeptor-spezifischer chimärer Antigenrezeptor gemäß einem der Ansprüche 1 bis 4, umfassend eine Spacer-Domäne zwischen der extrazellulären Domäne und der Transmembran-Domäne.

6. Gen, das den EPHB4-Rezeptor-spezifischen chimären Antigenrezeptor gemäß einem der Ansprüche 1 bis 5 codiert.

7. Verfahren zur Herstellung eines genmodifizierten Lymphozyten, der einen chimären Antigenrezeptor exprimiert, umfassend einen Schritt des Einführens des Gens gemäß Anspruch 6 in eine Zielzelle in vitro, vorzugsweise wobei das Einführen des Gens durch ein Transposonverfahren durchgeführt wird und die Zielzelle eine T-Zelle ist.

8. Verfahren zur Herstellung gemäß Anspruch 7, wobei das Transposonverfahren ein piggyBac-Transposonverfahren ist.

9. Genmodifizierter Lymphozyt, der den EPHB4-Rezeptor-spezifischen chimären Antigenrezeptor gemäß einem der Ansprüche 1 bis 5 exprimiert, wobei vorzugsweise der Lymphozyt eine T-Zelle ist.

10. Eine Zellpräparation, die eine therapeutisch wirksame Menge des genmodifizierten Lymphozyten gemäß Anspruch 9 umfasst.

11. Zellpräparation gemäß Anspruch 10 oder der genmodifizierte Lymphozyt gemäß Anspruch 9 zur Verwendung in einem Verfahren zur Behandlung eines Tumors oder Krebses, ausgewählt aus der Gruppe bestehend aus Rhabdomyosarkom, Lungenkrebs, Darmkrebs, malignem Mesotheliom, Speiseröhrenkrebs, Brustkrebs, Eierstockkrebs, Melanom und Kopf-Hals-Krebs.

12. Expressionskassette, die einen Promotor und das Gen gemäß Anspruch 6 unter der Kontrolle des Promotors umfasst.

13. Vektor mit der Expressionskassette gemäß Anspruch 12, wobei der Vektor vorzugsweise eine Struktur aufweist, bei der die Expressionskassette zwischen einem Paar von Transposon-Invertierten-Wiederholungssequenzen angeordnet ist.

14. Kit zur Herstellung eines genmodifizierten Lymphozyten, der einen EPHB4-Rezeptor-spezifischen chimären Antigenrezeptor exprimiert, wobei das Kit den Vektor gemäß Anspruch 13 und einen Transposase-Expressionsvektor umfasst, vorzugsweise wobei die Transposase eine piggyBac-Transposase ist.

15. Zellpräparation gemäß Anspruch 10 oder zur Verwendung gemäß Anspruch 11, die eine therapeutische Wirkung durch eine Zelltod-induzierende Wirkung durch EphrinB2-EPHB4-Bindung und eine zytotoxische Wirkung über einen chimären Antigenrezeptor ausübt.

## Revendications

1. Récepteur d'antigène chimérique spécifique au récepteur EPHB4 comprenant un domaine extracellulaire incluant un domaine extracellulaire d'EphrinB2, un domaine transmembranaire et un domaine de signal intracellulaire pour une fonction effectrice des immunocytes.

2. Récepteur d'antigène chimérique spécifique au récepteur EPHB4 selon la revendication 1, dans lequel le domaine extracellulaire d'EphrinB2 comprend une séquence d'acides aminés de SEQ ID NO : 1.

3. Récepteur d'antigène chimérique spécifique au récepteur EPHB4 selon la revendication 1 ou 2, dans lequel le domaine de signal intracellulaire comprend un domaine intracellulaire d'une molécule de costimulation et CD3.

4. Récepteur d'antigène chimérique spécifique au récepteur EPHB4 selon la revendication 3, dans lequel la molécule de costimulation est CD28.

5. Récepteur d'antigène chimérique spécifique au récepteur EPHB4 selon l'une quelconque des revendications 1 à 4, comprenant un domaine d'espacement entre le domaine extracellulaire et le domaine transmembranaire.

6. Gène codant pour le récepteur d'antigène chimérique spécifique au récepteur EPHB4 selon l'une quelconque des revendications 1 à 5.

7. Procédé de préparation d'un lymphocyte génétiquement modifié exprimant un récepteur d'antigène chimérique, comprenant une étape consistant à introduire le gène selon la revendication 6 dans une cellule cible in vitro, de préférence dans lequel l'étape consistant à introduire le gène est réalisée par un procédé de transposon, et la cellule cible est un lymphocyte T.

8. Procédé de préparation selon la revendication 7, dans lequel le procédé de transposon est un procédé de transposon piggyBac.

9. Lymphocyte génétiquement modifié exprimant le récepteur d'antigène chimérique spécifique au récepteur EPHB4 selon l'une quelconque des revendications 1 à 5, de préférence dans lequel le lymphocyte est un lymphocyte T.

10. Préparation cellulaire comprenant une quantité thérapeutiquement efficace du lymphocyte génétiquement modifié selon la revendication 9.

11. Préparation cellulaire selon la revendication 10 ou le lymphocyte génétiquement modifié selon la revendication 9 pour utilisation dans un procédé de traitement d'une tumeur ou d'un cancer choisi dans le groupe constitué de rhabdomyosarcome, le cancer du poumon, le cancer de l'intestin, le mésothéliome malin, le cancer de l'oesophage, le cancer du sein, le cancer de l'ovaire, le mélanome, et le cancer de la tête et du cou.

12. Cassette d'expression comprenant un promoteur et le gène selon la revendication 6 sous le contrôle du promoteur.

13. Vecteur ayant la cassette d'expression selon la revendication 12, de préférence dans lequel le vecteur a une structure dont la cassette d'expression est prise en sandwich entre une paire de séquences répétées inversées de transposons.

14. Kit pour la préparation d'un lymphocyte génétiquement modifié exprimant un récepteur d'antigène chimérique spécifique au récepteur EPHB4, le kit comprenant le vecteur selon la revendication 13 et un vecteur d'expression de transposase, de préférence dans lequel la transposase est la transposase piggyBac.

15. Préparation cellulaire selon la revendication 10 ou pour utilisation selon la revendication 11, qui exerce un effet thérapeutique par un effet d'induction de mort cellulaire par liaison EphrinB2-EPHB4 et un effet cytocide par l'intermédiaire d'un récepteur d'antigène chimérique.
